# EUROPEAN PATENT APPLICATION

(11) **EP 1 908 826 A1**
(43) Date of publication of application: **09.04.2008**
(21) Application number: 06767196.6
(22) Date of filing: 22.06.2006
(51) Int. Cl.: C12N 15/00, A61K 39/00, A61K 48/00, A61P 35/00, C07K 14/47, C12N 5/06, C12N 15/09

(54) **PROSTATE CANCER RELATED PROTEIN DERIVED PEPTIDE WHICH IS A PEPTIDE VACCINE CANDIDATE FOR PROSTATE CANCER PATIENTS WITH HLA-A3 SUPERTYPE ALLELES**

(30) Priority: 29.06.2005 JP 2005190105
(71) Applicant: Kurume University, Kurume-shi Fukuoka 8300011 (JP)
(72) Inventor: ITOH, Kyogo, 8410205 (JP); HARADA, Mamoru, 8130011 (JP); NOGUCHI, Masanori, 8300047 (JP)
(74) Representative: Vossius & Partner
(86) International application number: PCT/JP2006/312538
(87) International publication number: WO 2007/000935

(57) **Abstract**

According to the present invention, a peptide, which is a prostate-related protein derived peptide, capable of binding to an HLA-A3 supertype allele and recognized by the cellular and/or humoral immune system is provided. The peptides of the present invention make feasible the peptide-based anti-cancer vaccine therapy for prostate cancer patients with HLA-A3 supertype allele and useful for the treatment of prostate cancer patients with alleles other than *HLA-A2* and -*A24* to which cancer vaccine peptide candidates had been identified.

SEQUENCE LISTING

<110>  Green Peptide Co, Ltd.

<120>  Prostate cancer related protein derived peptide which is a peptide
vaccine candidate for prostate cancer patients with HLA-A3 supertype alleles

<130>  P1160 EP S3
<140>  06 76 7196.6
<141>  2006-06-22

<150>  JP 2005-190105
<151>  2005-06-29

<160>  46

<170>  PatentIn version 3.2

<210>  1
<211>  9
<212>  PRT
<213>  Artificial

<220>
<221>  source
<223>  /note="Description of artificaial sequence: PSA 104-112"

<400>  1

Ser Leu Leu Lys Asn Arg Phe Leu Arg
1               5


<210>  2
<211>  9
<212>  PRT
<213>  Artificial

<220>
<221>  source
<223>  /note="Description of artificaial sequence: PSA 60-68"

<400>  2

Val Leu Thr Ala Ala His Cys Ile Arg
1               5


<210>  3
<211>  9
<212>  PRT
<213>  Artificial

<220>
<221>  source
<223>  /note="Description of artificaial sequence: PSA 242-250"

<400>  3

Leu Tyr Thr Lys Val Val His Tyr Arg
1               5


<210>  4
<211>  9
<212>  PRT
<213>  Artificial

<220>
<221>  source
<223>  /note="Description of artificaial sequence: PSA 16-24"

<400>  4

Gly Ala Ala Pro Leu Ile Leu Ser Arg
1               5


<210>  5
<211>  10
<212>  PRT
<213>  Artificial

<220>
<221>  source
<223>  /note="Description of artificaial sequence: PSA 241-250"

<400>  5

Ser Leu Tyr Thr Lys Val Val His Tyr Arg
1               5                   10


<210>  6
<211>  10
<212>  PRT
<213>  Artificial

<220>
<221>  source
<223>  /note="Description of artificaial sequence: PSA 59-68"

<400>  6

Trp Val Leu Thr Ala Ala His Cys Ile Arg
1               5                   10


<210>  7
<211>  10
<212>  PRT
<213>  Artificial

<220>
<221>  source
<223>  /note="Description of artificaial sequence: PSA 36-45"

<400>  7

Gln Pro Trp Gln Val Leu Val Ala Ser Arg
1               5                   10


<210>  8
<211>  10
<212>  PRT
<213>  Artificial

<220>
<221>  source
<223>  /note="Description of artificaial sequence: PSA 192-201"

<400>  8

Val Thr Lys Phe Met Leu Cys Ala Gly Arg
1               5                   10

<210>  9
<211>  10
<212>  PRT
<213>  Artificial

<220>
<221>  source
<223>  /note="Description of artificaial sequence: PSA 100-109"

<400>  9

Leu Tyr Asp Met Ser Leu Leu Lys Asn Arg
1               5                   10


<210>  10
<211>  10
<212>  PRT
<213>  Artificial

<220>
<221>  source
<223>  /note="Description of artificaial sequence: PSA 68-77"

<400>  10

Arg Asn Lys Ser Val Ile Leu Leu Gly Arg
1               5                   10


<210>  11
<211>  9
<212>  PRT
<213>  Artificial

<220>
<221>  source
<223>  /note="Description of artificaial sequence: PAP 20-28"

<400>  11

Phe Leu Leu Phe Phe Trp Leu Asp Arg
1               5


<210>  12
<211>  9
<212>  PRT
<213>  Artificial

<220>
<221>  source
<223>  /note="Description of artificaial sequence: PAP 324-332"

<400>  12

Glu Tyr Phe Val Glu Met Tyr Tyr Arg
1               5


<210>  13
<211>  9
<212>  PRT
<213>  Artificial

<220>

<221>  source
<223>  /note="Description of artificaial sequence: PAP 39-47"

<400>  13

Thr Leu Val Phe Arg His Gly Asp Arg
1               5


<210>  14
<211>  9
<212>  PRT
<213>  Artificial

<220>
<221>  source
<223>  /note="Description of artificaial sequence: PAP 78-86"

<400>  14

His Tyr Glu Leu Gly Glu Tyr Ile Arg
1               5


<210>  15
<211>  9
<212>  PRT
<213>  Artificial

<220>
<221>  source
<223>  /note="Description of artificaial sequence: PAP 155-163"

<400>  15

Tyr Leu Pro Phe Arg Asn Cys Pro Arg
1               5


<210>  16
<211>  10
<212>  PRT
<213>  Artificial

<220>
<221>  source
<223>  /note="Description of artificaial sequence: PAP 96-105"

<400>  16

Ser Tyr Lys His Glu Gln Val Tyr Ile Arg
1               5                   10


<210>  17
<211>  10
<212>  PRT
<213>  Artificial

<220>
<221>  source
<223>  /note="Description of artificaial sequence: PAP 171-180"

<400>  17

Thr Leu Lys Ser Glu Glu Phe Gln Lys Arg
1               5                   10

<210>  18
<211>  10
<212>  PRT
<213>  Artificial

<220>
<221>  source
<223>  /note="Description of artificaial sequence: PAP 19-28"

<400>  18

Leu Phe Leu Leu Phe Phe Trp Leu Asp Arg
1               5                   10


<210>  19
<211>  10
<212>  PRT
<213>  Artificial

<220>
<221>  source
<223>  /note="Description of artificaial sequence: PAP 38-47"

<400>  19

Val Thr Leu Val Phe Arg His Gly Asp Arg
1               5                   10


<210>  20
<211>  10
<212>  PRT
<213>  Artificial

<220>
<221>  source
<223>  /note="Description of artificaial sequence: PAP 102-111"

<400>  20

Val Tyr Ile Arg Ser Thr Asp Val Asp Arg
1               5                   10


<210>  21
<211>  10
<212>  PRT
<213>  Artificial

<220>
<221>  source
<223>  /note="Description of artificaial sequence: PAP 154-163"

<400>  21

Leu Tyr Leu Pro Phe Arg Asn Cys Pro Arg
1               5                   10


<210>  22
<211>  10
<212>  PRT
<213>  Artificial

<220>
<221>  source

<223>  /note="Description of artificaial sequence: PAP 248-257"

<400>  22

Gly Ile His Lys Gln Lys Glu Lys Ser Arg
1               5                   10


<210>  23
<211>  9
<212>  PRT
<213>  Artificial

<220>
<221>  source
<223>  /note="Description of artificaial sequence: PSMA 590-598"

<400>  23

Ser Ile Val Leu Pro Phe Asp Cys Arg
1               5


<210>  24
<211>  9
<212>  PRT
<213>  Artificial

<220>
<221>  source
<223>  /note="Description of artificaial sequence: PSMA 173-181"

<400>  24

Asp Leu Val Tyr Val Asn Tyr Ala Arg
1               5


<210>  25
<211>  9
<212>  PRT
<213>  Artificial

<220>
<221>  source
<223>  /note="Description of artificaial sequence: PSMA 199-207"

<400>  25

Lys Ile Val Ile Ala Arg Tyr Gly Lys
1               5


<210>  26
<211>  9
<212>  PRT
<213>  Artificial

<220>
<221>  source
<223>  /note="Description of artificaial sequence: PSMA 680-688"
<400>  26

Gly Leu Pro Asp Arg Pro Phe Tyr Arg
1               5


<210>  27

<211>  9
<212>  PRT
<213>  Artificial

<220>
<221>  source
<223>  /note="Description of artificaial sequence: PSMA 370-378"

<400>  27

Arg Tyr Val Ile Leu Gly Gly His Arg
1               5


<210>  28
<211>  9
<212>  PRT
<213>  Artificial

<220>
<221>  source
<223>  /note="Description of artificaial sequence: PSMA 455-463"

<400>  28

Ser Ile Glu Gly Asn Tyr Thr Leu Arg
1               5


<210>  29
<211>  9
<212>  PRT
<213>  Artificial

<220>
<221>  source
<223>  /note="Description of artificaial sequence: PSMA 403-411"

<400>  29

Gly Thr Leu Lys Lys Glu Gly Trp Arg
1               5


<210>  30
<211>  9
<212>  PRT
<213>  Artificial

<220>
<221>  source
<223>  /note="Description of artificaial sequence: PSMA 247-255"

<400>  30

Asn Leu Pro Gly Gly Gly Val Gln Arg
1               5


<210>  31
<211>  9
<212>  PRT
<213>  Artificial

<220>
<221>  source
<223>  /note="Description of artificaial sequence: PSMA 641-649"

<400>  31

Glu Ile Ala Ser Lys Phe Ser Glu Arg
1               5


<210>  32
<211>  9
<212>  PRT
<213>  Artificial

<220>
<221>  source
<223>  /note="Description of artificaial sequence: PSMA 207-215"

<400>  32

Lys Val Phe Arg Gly Asn Lys Val Lys
1               5


<210>  33
<211>  10
<212>  PRT
<213>  Artificial

<220>
<221>  source
<223>  /note="Description of artificaial sequence: PSMA 354-363"

<400>  33

Arg Ile Tyr Asn Val Ile Gly Thr Leu Arg
1               5                   10


<210>  34
<211>  10
<212>  PRT
<213>  Artificial

<220>
<221>  source
<223>  /note="Description of artificaial sequence: PSMA 181-190"

<400>  34

Arg Thr Glu Asp Phe Phe Lys Leu Glu Arg
1               5                   10


<210>  35
<211>  10
<212>  PRT
<213>  Artificial

<220>
<221>  source
<223>  /note="Description of artificaial sequence: PSMA 675-684"

<400>  35

Phe Ile Asp Pro Leu Gly Leu Pro Asp Arg
1               5                   10


<210>  36
<211>  10

<212>  PRT
<213>  Artificial

<220>
<221>  source
<223>  /note="Description of artificaial sequence: PSMA 525-534"

<400>  36

Phe Gln Arg Leu Gly Ile Ala Ser Gly Arg
1               5                   10


<210>  37
<211>  10
<212>  PRT
<213>  Artificial

<220>
<221>  source
<223>  /note="Description of artificaial sequence: PSMA 345-354"

<400>  37

His Ile His Ser Thr Asn Glu Val Thr Arg
1               5                   10


<210>  38
<211>  10
<212>  PRT
<213>  Artificial

<220>
<221>  source
<223>  /note="Description of artificaial sequence: PSMA 201-210"

<400>  38

Val Ile Ala Arg Tyr Gly Lys Val Phe Arg
1               5                   10


<210>  39
<211>  10
<212>  PRT
<213>  Artificial

<220>
<221>  source
<223>  /note="Description of artificaial sequence: PSMA 361-370"

<400>  39

Thr Leu Arg Gly Ala Val Glu Pro Asp Arg
1               5                   10


<210>  40
<211>  10
<212>  PRT
<213>  Artificial

<220>
<221>  source
<223>  /note="Description of artificaial sequence: PSMA 571-580"

<400>  40

Lys Tyr His Leu Thr Val Ala Gln Val Arg
1               5                   10

<210>  41
<211>  10
<212>  PRT
<213>  Artificial

<220>
<221>  source
<223>  /note="Description of artificaial sequence: PSMA 272-281"

<400>  41

Tyr Pro Ala Asn Glu Tyr Ala Tyr Arg Arg
1               5                   10

<210>  42
<211>  10
<212>  PRT
<213>  Artificial

<220>
<221>  source
<223>  /note="Description of artificaial sequence: PSMA 431-440"

<400>  42

Ser Thr Glu Trp Ala Glu Glu Asn Ser Arg
1               5                   10

<210>  43
<211>  9
<212>  PRT
<213>  Artificial

<220>
<221>  source
<223>  /note="Description of artificaial sequence: EBV-derived peptide"

<400>  43

Ile Val Thr Asp Phe Ser Val Ile Lys
1               5

<210>  44
<211>  10
<212>  PRT
<213>  Artificial

<220>
<221>  source
<223>  /note="Description of artificaial sequence: Flu-derived peptide"

<400>  44

Asn Val Lys Asn Leu Tyr Glu Lys Val Lys
1               5                   10

<210>  45
<211>  9
<212>  PRT

<213>  Artificial

<220>
<221>  source
<223>  /note="Description of artificaial sequence: TRP2-derived peptide"

<400>  45

Leu Leu Gly Pro Gly Arg Pro Tyr Arg
1               5


<210>  46
<211>  11
<212>  PRT
<213>  Artificial

<220>
<221>  source
<223>  /note="Description of artificaial sequence: HIV-derived peptide"

<400>  46

Arg Leu Arg Asp Leu Leu Leu Ile Val Thr Arg
1               5                   10

## Description

### TECHNICAL FIELD

The present invention relates to peptides which are derived from prostate cancer related protein and are useful for the treatment of prostate cancer in a patient with human histocompatibility leukocyte antigen (HLA)-A3 supertype alleles.

### BACKGROUND ART

Prostate cancer is one of the most common cancers among elderly men (Non Patent Literature 1). Despite the fact that androgen withdrawal therapy is transiently effective for prostate cancer, there is no effective therapy against recurrent hormone-refractory, and bone metastatic prostate cancer. For such patients, specific immunotherapy may be a promising option because prostate cancer-reactive citotoxic T cells could detect multiple metastases with fine specificity. So far, many cancer-related antigens and their peptides that are recognized by CTLs have been identified (Non patent literature 2). In addition, several antigenic peptides derived from prostate-specific antigen (PSA) (Non Patent Literatures 3-5), prostate-specific membrane antigen (PSMA) (Non patent literatures 6, 7), prostatic acid phosphatase (PAP) (Non patent literatures 8, 9) or prostate stem cell antigen (PSCA) (Non patent Literatures 10-12) have been identified. As is the case with melanocyte differentiation antigens for melanoma, these prostate-related antigens have been considered to be promising targets in specific immunotherapy for prostate cancer patients (Non patent Literature 13). However, the peptide vaccine candidates identified to date have been focused on the HLA-A2 and -A24 alleles, because of the higher worldwide frequency of these alleles.

Based on the structural similarities of the group of HLA alleles and the peptide binding motif analysis, the following supertypes have been proposed: HLA-A2, -A3, -B7, and -B44 supertype alleles (Non Patent Literature 14). Among them, the A3 supertype allele is found in 38% of Caucasians, 53% of Chinese, 46% of Japanese, and 43% of North American African-Americans and Hispanics (Non Patent Literature 14).

### REFERENCES

[Non Patent Literature 1] Greenlee RT, Murray T, Bolden S, Wingo PA.. Cancer statistics, 2000. CA Cancer J Clin 2000;50:7-33.

[Non Patent Literature 2] Renkvist N. Castelli C, Robbins PF, Parmiani G. A listing of human tumor antigens recognized by T cells. Cancer Immunol Immunother 2001;50:3-15.

[Non Patent Literature 3] Correale P, Walmsley K, NIeroda C, et al. In vitro generation of human cytotoxic T lymphocytes specific for peptides derived from prostate-specific antigen. J Natl Cancer Inst 1997;89:293-300.

[Non Patent Literature 4] Xue BH, Zhang Y, Sosman J, Peace DJ. Induction of human cytotoxic T lymphocytes specific for prostate-specific antigen. Prostate 1997;30:73-78.

[Non Patent Literature 5] Correale P, Walmsley K, Zaremba S, Zhu MZ, Schlom J, Tsang KY. Generation of human cytotoxic T lymphocyte lines directed against prostate-specific (PSA) employing a PSA oligoepitope peptide. J Immunol 1998;161:3186-94.

[Non Patent Literature 6] Horiguchi Y, Nukaya I, Okazawa K, et al. Screening of HLA-A24-restrlcted epitope peptides from prostate-specific membrane antigen that induce specific antitumor cytotoxic T lymphosytes. Clin Cancer Res 2002;8:3885-92.

[Non Patent Literature 7] Tjoa B, Kenny G, Ragde H, Misrock SL, Murphy G. Presentation of prostate tumor antigens by dendritic cells stimulates T-cell proliferation and cytotoxicity. Prostate 1996;28:65-9.

[Non Patent Literature 8] Inoue Y, Takaue Y, Takei M, et al. Induction of tumor specific cytotoxic T lymphocytes in prostate cancer using prostatic acid phosphatase derived HLA-A2402 bindin peptide. J Urol 2001;166:1508-13.

[Non Patent Literature 9] Peshwa MV, Shi JD, Ruegg C, Laus R, van Schooten WC. Induction of prostate tumor-specific CD8+ cytotoxic T-pymphocytes in vitro usin antigen-presenting cells pulsed with prostatic acid phosphatase peptide. Prostate 1998;36:129-38.

[Non Patent Literature 10] Dannull J, Diener PA, Prikler L, et al. Prostate stem cell antigen is a promising candidate for immunotherapy of advanced prostate cancer Cancer Res 2001;60:5522-8.

[Non Patent Literature 11] Matsueda S, Kobayashi K, Nonaka Y, Noguchi M, Itoh K, Harada M. Identification of new prostate stem cell antigen-derived peptides immunogenic in HLA-A2(+) patients with hormone-refractory prostate cancer. Cancer Immunol Immunother 2004;53:479-89.

[Non Patent Literature 12] Matsueda S, Yao A, Ishihara Y, et al. A prostate stem cell antigen-derived peptide immunogenic in HLA-A24+ prostate cancer patients. Prostate 2004;60:205-13.

[Non Patent Literature 13] Harada M, Noguchi M, Itoh K. Target molecules in specific immunotherapy against prostate cancer. Int J Clin Oncol 2003; 8:193-9.

[Non Patent Literature 14] Sette, A., and Sidney, J. Ninemajor HLA class supertypes account for the vast preponderance of HLA-A and -B polymorphism. Immunogenetics, 50:201-212, 1999.

### DISCLOSURE OF THE INVENTION

### PROBLEM TO BE SOLVED BY THE INVENTION

An object of the present invention is to provide prostate relating protein derived peptides which expands the possibility of the treatment of prostate cancer patients.

### MEANS FOR SOLVING THE PROBLEM

The present invention provides a peptide which is derived from a prostate cancer related protein, capable of binding to an HLA-A3 supertype allele and recognized by the cellular and/or humoral immune system. In particular, the present invention provides a peptide having an amino acid sequence shown in any one of SEQ ID NOS: 4, 15, 22, 32 and 42 or a derivative thereof which has the functionally equivalent properties. The present invention also provides a nucleic acid molecule encoding the peptide or derivative thereof of the present invention, and a vector comprising the nucleic acid molecule.

The present invention further provides a pharmaceutical composition, especially the composition which is a cancer vaccine, for the treatment or prevention of prostate cancer.

The present invention further provides a method for the treatment or prevention of prostate cancer, which comprises administering the peptide, peptide derivative or vector of the present invention to a subject to be treated. According to the method of the present invention, the peptide, peptide derivative or vector is administered as cancer vaccine.

The present invention also provide use of the peptide, peptide derivative or vector of the present invention for the manufacture of a pharmaceutical composition, especially a cancer vaccine, for the treatment or prevention of prostate cancer.

The present invention further provides a method for inducing prostate caner reactive cytotoxic T cell, which comprises contacting peripheral blood mononuclear cell isolated from a prostate cancer patient with an HLA-A3 supertype allele with the peptide or peptide derivative of the present invention.

Further more, the present invention provides a method for the preparation of an antigen presenting cell which presents a complex between the prostate cancer related protein derived peptide or a derivative thereof and an HLA-A3 supertype allele on the surface of the cell, which comprises allowing a cell having antigen-presenting ability isolated from a prostate cancer patient with an HLA-A3 supertype allele to be incorporated with the peptide, peptide derivative or vector of the present invention.

According to the present invention, peptide-based anti-cancer immune therapy, especially, cancer vaccine therapy for prostate patients with HLA-A3 supertype alleles became possible. The instant invention is especially useful for the treatment of an HLA-A2 and -A24 negative prostate cancer patient. Some peptide vaccine candidates for a patient with HLA-A2 or -A24 had been identified to date.

### BRIEF DESCRIPTION OF THE DRAWINGS

[Figure 1] Figure 1 provides graphs showing the expression of HLA-A3 supertype allels on LNCaP sublines. The unshaded portion of the graph represents staining without the first mAb.
[Figure 2] Figure 2 provides graphs showing specificity of peptide-reactive IgGs. The levels of IgGs reactive to the peptides in the plasma of patients were significantly diminished by incubating the samples in wells that were coated with each of the corresponding peptides.
[Figure 3] Figure 3 provides graphs showing cytotoxicity of peptide-stimulated peripheral blood mononuclear cells (PBMCs) from HLA-A3 supertype+ prostate cancer patients and healthy donors. PBMCs from HLA-A3 supertype+ prostate cancer patients and healthy donors stimulated with the peptides indicated in the graphs exhibited high cytotoxicity against prostate cancer cell line that expressing corresponding HLA.
[Figure 4] Figure 4 provides graphs showing that the cytotoxicity of peptide-stimalated PBMCs against prostate cancer cells is Class I-restricted and CD8+ T cell-dependent. The cytotoxicity of peptide-stimulated PBMCs was significantly inhibited by the addition of anti-HLA Class-1 mAb.
[Figure 5] Figure 5 provides graphs showing that the cytotoxicity against prostate cancer cells was dependent on peptide-specific CTLs. The cytotoxicity of the peptide-stimulated PBMCs was significantly inhibited by the addition of cold target cells stimulated with the corresponding peptide.
[Figure 6] Figure 6 provides graphs showing cytotoxicity of peptide stimulated PBMCs against prostate cancer cells expressing various HLA-A3 supertype alleles. The PBMCs from patients expressing each of HLA-A3 supertype alleles that were stimulated with the peptides indicated in the graphs were cytotoxic against prostate cancer cells positive for each of the HLA-A3 supertype alleles.

### [BEST MODE FOR CARRYING OUT THE INVENTION]

The peptide of the invention is a peptide fragment of a prostate related protein. In the instant invention, "prostate related protein" refers to prostate-specific antigen (PSA), prostate-specific membrane antigen (PSMA) or prostatic acid phosphatase (PAP). The amino acid sequences of those proteins have been disclosed by GenBank under accession numbers: M26663 (PSA); AF007544 (PSMA); and M24902(PAP).

According to the invention, the phrase "a peptide is capable of binding to an HLA-A3 supertype allele" means that said peptide binds to an HLA-A3 supertype allele to form complex and the complex is presented on the cell surface. In general, peptides that are capable of binding to an HLA molecule shares some specific amino acid sequences depending on the types of the HLA. The specific amino acid sequences are called as "binding motifs". HLA-A3 supertype alleles comprise HLA-A11, -A31, -A33, -A0301 and -A6801 alleles and all of them share the binding motifs (Sette, A., and Sidney, J. Ninemajor HLA class I supertypes account for the vast preponderance of HLA-A and -B polymorphism. Immunogenetics, 50:201-212, 1999.). Peptides having the binding motif to the HLA-A3 supertype alleles can be determined using computer analysis such as Bioinformatics and Molecular Analysis Section (NIH, Bethesda, MD).

According to the instant application, the phrase "a peptide is recognized by the cellular immune system" means that the peptide is recognized by specific CTL. In other word, the peptide has an ability to induce the peptide specific CTL. The art can determine whether or not a peptide is recognized by CTL by a known method. For example, by determining whether or not a cytokine such as γ-IFN is produced by CTL in response to antigen presenting cells which are pulsed with said peptide using the ELISA technique. In addition, cytotoxic activity of the induced CTL can be determined by the ⁵¹Cr-release assay and the like. Preferred length of the amino acid sequence of the peptide of the present invention is 8 to 14, more preferably 8 to 11 and especially, 9 or 10 amino acid residues in view of good recognition by CTL.

According to the invention, the phrase "a peptide is recognized by the humoral immune system" means that an IgG specific to said peptide is present in the body. That is, the peptide-specific IgG is detected in the plasma of the subject. The inventors had previously reported that IgGs reactive to the CTL epitope peptides were frequently detected in the plasma of prostate cancer patients (Nakatsura T, Senju S, Ito M, Nishimura Y, Itoh K., Eur J Immunol. 2002;32:826-36.;Ohkouchi S, Yamada A, Imai N, et al., Tissue Antigens 2002;59:259-72), and that IgGs reactive to PSA or PSMA derived CTL oriented peptide can be detected in the plasma of prostate cancer patients as well as healthy people (Harada M, Kobayashi K, Matsueda S, Nakagawa M, Noguchi M, Itoh K. Prostate 2003;57:152-9.; Kobayashi K, Noguchi M, Itoh K, Harada M., Cancer Science 2003:94:622-7.). Peptides that are frequently recognized by the plasma IgGs are expected to have an ability to induce peptide specific CTL. The amount of the specific IgGs can be determined by commonly known ELISA techniques and the like.

Peptides recognized by both the cellular and humoral immune systems are expected to exhibits higher immunogenicity in the body and therefore, are preferable as peptides of the present invention. A peptide consisting of an amino acid sequence shown in any one of Sequence ID Nos. 4, 15, 22, 32 and 42 is especially useful.

According to the instant invention, "peptide derivative" of the invention is a derivative of the peptide of Sequence ID Nos.4, 15, 22, 32 or 42 and has one or two substitution in the original amino acid sequence, and/or deletion and/or addition of one or two amino acids to the original amino acid sequence in the range so that the derivative has 8-11 amino acid residues. The phrase "peptide derivative has the functionally equivalent properties" means that the peptide derivative is capable of binding to an HLA-A3 supertype allele and recognized by the cellar and/or humoral immune system. Whether or not a peptide derivative has the functionally equivalent properties can be determined by the above-described procedures.

In order to do not alter the property of the original peptide, the substitution of amino acid residue is preferably made within the amino acids belonging to the same group, such as polar amino acids, non-polar amino acids, hydrophobic amino acids, hydrophilic amino acids, positively charged amino acids, negatively charged amino acids and aromatic amino acids. In addition, the substitution, deletion and/or addition of amino acid is preferably made so that the derivative is acceptable in view of HLA binding motifs. That is, the C-terminus amino acid of the peptide derivative is preferably lysine or arginine. According to the instant invention, peptide derivatives of SEQ ID Nos. 4, 15, 22, 32 or 42 whose C-terminus amino acid residue is substituted by lysine or arginine is especially preferable.

The amino acid constituting the peptides and peptide derivatives of the invention may be natural amino acids or amino acid analogues. Amino acid analogus may include N-acylated, O-acylated, esterified, acid amidate and alkylated amino acids. The amino or carboxylic group or the like of the amino acid residue, constituting the peptide or a derivative thereof may be modified so long as it does not significantly deteriorate the function of the peptide. The modification may be addition of formyl, acetyl or t-butoxycarbonyl at the N-terminus- or free-amino group, or addition of methyl, ethyl, t-butyl or benzyl group at the C-terminus- or free carboxylic group.

The peptide and peptide derivative according to the instant invention may be synthesized by a conventionally used peptide synthesizing procedure. Examples of the conventionally used procedures are those described in the literatures including "Peptide Synthesis", Interscience, New York, 1966; "The Proteins", vol. 2, Academic Press Inc., New York, 1976; *"*Pepuchido-Gosei", Maruzen Co. Ltd., 1975; *"*Pepuchido-Gosei-no-Kiso-to-Jikkenn", Maruzen Co. Ltd., 1985; and *"*Iyakuhin-no-Kaihatu, Zoku, vol. 14, Peputido-Gosei", Hirokawa Shoten, 1991.

The peptide and peptide derivative of the present invention may be those generated by fragmentation of a peptide containing the amino acid sequence of the peptide or peptide derivative of the present invention in a cell and provided as complex with the HLA molecule. The instant invention encompass the use of such peptide as above. As long as the peptide or peptide derivative of the present invention can be provided, the peptide may comprise any number of amino acid residues.

The peptide and peptide derivative of the present invention can effectively induce and growth CTLs which are toxic to HLA-A3 supertype allel positive prostate cancer cells. Accordingly, the peptide and peptide derivative of the present invention can be used for inducing prostate cancer reactive CTL and for manufacturing pharmaceutical composition against prostate cancer and therefore, useful for the treatment of prostate cancer.

The pharmaceutical composition of the present invention comprises one or more of the peptide or peptide derivative of the present invention. The peptide or derivative can treat the prostate cancer by inducing peptide-specific CTL which is reactive to prostate cancer. The pharmaceutical composition of the present invention can be used as a cancer vaccine. Since CTL of a patient is an aggregate of the cells recognizing a plurality of different cancer antigen peptides, it is effective to use a plurality of the peptides or peptide derivatives of the present invention in combination. The peptide or peptide derivative of the invention may be used in combination with a cancer antigen peptide other than the peptide of the present invention. The pharmaceutical composition of the present invention may be administered along with an adjuvant which has conventionally been used for vaccination in order to establish the immunity effectively. In addition, the pharmaceutical composition of the present invention may be formulated as liposomal preparations, particulate preparations in which the ingredient is bound to beads having a diameter of several micro maters, or preparations in which the ingredient is attached to lipids.

The pharmaceutical composition of the invention may be administered, for example, intradermally or subcutaneously. The amount of the peptide or peptide derivative to be administered may be determined based on the condition of the disease to be treated, age and body weight of the respective patient. The amount of the peptide or peptide derivative of the present invention in a dosage form may be 0.0001mg-1000mg, preferably 0.0001mg-100mg, more preferably 0.001mg-10mg. The dosage form may preferably be administered once every several days, several weeks or several months for 1-3 years.

The nucleic acid molecule of the present invention can provide the peptide or peptide derivative of the present invention. By introducing a vector in which the nucleic acid molecule of the invention is incorporated in an antigen presenting cell and expressing the same, a complex between the HLA and the peptide or peptide derivative of the present invention is expressed on the surface of the cell. Thus obtained antigen presenting cell can effectively growth peptide specific prostate cancer reactive CTLs. In the case the nucleic acid molecule of the present invention is used for the treatment of prostate cancer, the vector comprising the nucleic acid molecule is administered to the patient so that the vector is expressed in the body of the patient, or the vector is introduced *ex vivo* in a suitable cell, for example a dendric cell isolated from the patient, and then the cell is returned to the patient. Those methods are well known in the art (Hrouda D, Dalgleish AG. Gene therapy for prostate cancer. Gene Ther 3: 845-52, 1996).

Examples of vectors in which the nucleic acid molecule of the present invention is incorporated may include various plasmid vectors and viral vectors such as adenovirus, adeno-associated virus, retrovirus and vaccinia virus vectors(Liu M, Acres B, Balloul JM, Bizouarne N, Paul S, Slos P, Squiban P. Gene-based vaccines and immunotherapeutics. Proc Natl Acad Sci U S A 101 Suppl, 14567-71, 2004) . Methods for preparing vectors have been well known in the art (Molecular Cloning: A laboratory manual, 2nd ed. New York, Cold Spring Harbor Laboratory) .

The vector of the present invention can be formulated as a pharmaceutical composition for the treatment or prevention of prostate cancer. The amount of the vector to be administered may vary depending on the condition of the disease to be treated, the age and body weight of the patient to be treated and the like, and may preferably be 0.1µg-100mg, more preferably 1µg-50mg as an amount of DNA. The pharmaceutical composition may be administered be administered, for example, intravenously, subcutaneously, or intradermally.

As is apparent from the above description, the peptide or peptide derivative of the present invention as well as the vector of the present invention can be used for the method for the treatment or prevention of prostate cancer, and also for the manufacture of a pharmaceutical composition for the treatment or prevention of prostate cancer.

By the method for introducing CTL according to the present invention, CTLs against HLA-A3 supertype allele positive prostate cancer cells are provided. In the instant invention, "prostate cancer reactive CTL" refers the property of the CTL which can recognize the complex between the cancer antigen peptide and the HLA molecule on the prostate cancer cells and kill the recognized cells. The method of inducing CTLs according to the present invention may be carried out for example by incubating the PBMC isolated from an HLA-A3 supertype allele positive prostate cancer patient *in vitro* in the presence of the peptide or peptide derivative of the present invention. CTLs induced by the instant method are useful for the adoptive immunotherapy, i.e. for treating the cancer by returning the same into the patient from which the PBMC is isolated so that the CTLs kill the cancer cells. That is, the CTL of the present invention is useful as a pharmaceutical composition for the treatment or prevention of prostate cancer.

The CTL inducing kit of the present invention can be used for the aforementioned method for inducing CTL. The kit of the present invention comprises one or more peptide or peptide derivative of the present invention. In addition, the kit may further comprises a suitable buffer, culture media and the like.

By the method for the preparation of antigen presenting cells of the present invention, antigen presenting cells which can be used for inducing CTL against HLA-A3 supertype allele positive prostate cancer cells are provided. The method of the preparation of antigen presenting cells of the present invention, for example, may be carried out by pulsing cells having antigen-presenting ability isolated from the HLA-A3 supertype allele positive prostate cancer patient with the peptide or peptide derivative of the present invention so that the cells incorporate the peptide, or by introducing the vector of the present invention into said cells in a conventional manner. The cells having antigen presenting ability may be, for example, dendritic cells which can be prepared from PBMC obtained from the patient by isolating the cells adhered to the culture plate of the PBMC culture and then, incubating the isolated cells in the presence of IL-4 and GM-CSF for one week. The antigen presenting cells prepared by the method of the present invention can induce CTLs that specifically recognize the complex between the peptide or peptide derivative of the present invention and the HLA molecule presented on the surface of the cells. When the antigen presenting cells of the invention are administered to the prostate cancer patient, they can induce prostate cancer reactive CTLs in the body of the patient. Accordingly, the antigen presenting cell of the present invention can be used as a pharmaceutical composition for the treatment or prevention of prostate cancer.

The kit for the preparation of antigen presenting cells according to the present invention is used for carrying out the aforementioned method of the present invention. The kit of the present invention comprise one or more of the peptide or peptide derivative of the present invention and may further comprise a suitable buffer, culture media and the like.

### EXAMPLES

The present invention is further illustrated by the following examples, but is not restricted by these examples in any way.

### 1. METHOD

### 1.1 Patients.

PBMCs were obtained from HLA-A3 supertype⁺ prostate cancer patients including HLA-A11⁺ (n = 5), -A31⁺ (n = 5), and -A33⁺ (n = 5 ) patients. The patients had provided written informed consent. PBMCs from HLA-A3⁺ or - A68.1⁺ patients were not available because of their extremely low frequency (1.6% and 0.5%) in the Japanese population (Aizawa M. The Proceedings of the 3rd Asia-Oceania Histocompaatibility Workshop Conference, pp. 1090-1103. Oxford: Oxford University Press, 1986.). None of the participants was infected with HIV. Twenty ml of peripheral blood was obtained from the patient, and PBMCs were prepared by the Ficoll-Conray density gradient centrifugation. All of the samples were cryopreserved until they were used for the experiments. The expression of HLA-A11, -A31, and -A33 molecules on PBMCs of cancer patients was determined by flow cytometry using the following antibodies: anti-HLA-A11 monoclonal antibody EmAb)(Cat# 0284HA; One Lambda Inc., Canoga, CA), anti-HLA-A31 mAb (Cat# 0273HA; One Lambda), and anti-HLA-A33 mAb (Cat# 0612HA; One Lambda). This study protocol was approved by ethical review boards of the Kurume University School of Medicine, and the approved the study protocol.

### 1.2 Cell Lines.

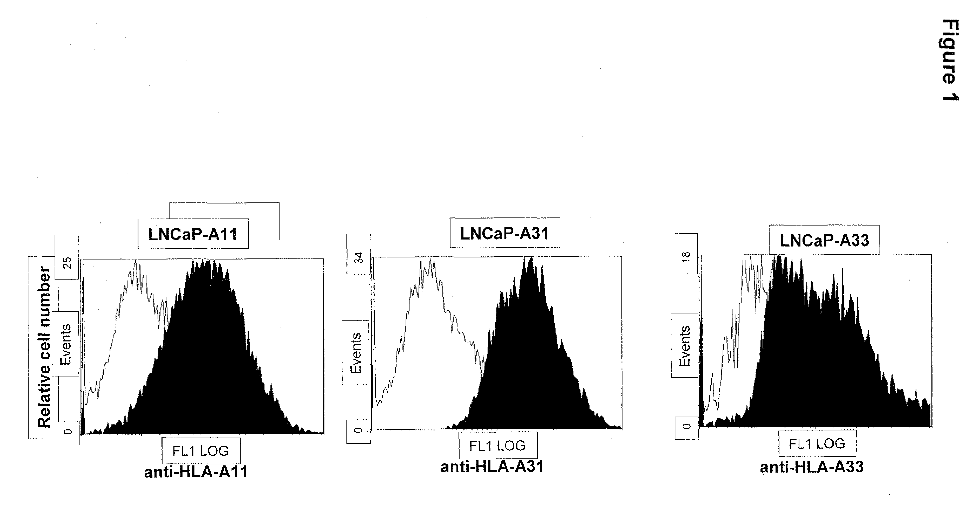

C1R is a B lymphoblastoid cell line which is suitable for HLA-class I molecular gene introduction (CRL-1993). C1R-A11, -A31, and -A33 are sublines that were stably transfected with the *HLA*-*A1101,* -*A3101*, and *-A3303* gene, respectively. The expressions of HLA-A11, -A31, and - A33 molecules on these sublines were previously reported (Takedatsu H, Shichijo S, Katagiri K, Sawamizu H, Sata M, Itoh K., Clinical Cancer Reseach 2004;10:1112-20.). LNCaP is an HLA-A*0201⁺ prostate carcinoma cell line(CRL-1740). To generate LNCaP sublines expressing each of the KLA-A11, -A31, and -A33 molecules, an *HLA-A1101, -A3101,* or *-A3303* plasmid cDNA was inserted into the eukaryotic expression vector pCR3.1 (Invitrogen, Carlsbad, CA) by a method reported previously (Yang D, Nakao M, Shichijo S, et al., Cancer Res 1999;59:4056-63.). Electroporatin was performed using a Gene Pulser (Bio RAD, Richmond, CA). LNCaP-A11, - A31, and -A33 are sublines that were stably transfected with the *HLA-A1101, -A3101,* and *-A3303* genes, respectively (Figure 1). All of the cell lines were maintained in RPMI 1640 (Invitrogen) with 10% FCS.

### 1.3 Induction of Peptide-Specific CTLs from PBMCs.

Assays for the detection of peptide-specific CTLs were performed according to a previously reported method with several modifications (Hida N, Maeda Y, Katagiri K, Takasu H, Harada M, Itoh K., Cancer Immunol Immunother 2002;51:219-28.). In brief, PBMCs were stimulated with peptides derived from PSA, PAP and PSMA, and the control peptide. The amount of IFN-gamma generated in response to the C1R-A11, C1R-A31 or C1RA33 cells pulsed with corresponding peptide was determined. In brief, PBMC (1×10⁵ cells/well) were incubated with 10 µl/ml of each peptide in quadruplicate in a U-bottom-type 96-well microculture plate (Nunc, Roskilde, Denmark) in 200 µl of culture medium. The culture medium consisted of 45% RPMI 1640, 45% AIM-V medium (Gibco-BRL, Gaithersburg, MD), 10% FCS, 100 U/ml of interleukin-2 (IL-2), and 0.1 mM MEM nonessential amino acid solution (Gibco-BRL). Half of the culture medium was removed and replaced with new medium containing a corresponding peptide (10 µg/ml) every 3 days. On the 15^{th} day of culture, half of the cultured cells were stimulated with the corresponding peptide-pulsed C1R-A11, - A31, or -A33 cells, and the other half of the cells were cultured C1R-A11, -A31, or -A33 cells pulsed with the HIV derived peptide(control peptide). After an 18-hr incubation, the supernatant was collected, and the level of interferon (IFN)-γ was determined by enzyme-linked immunosorbent assay (ELISA). The successful induction of peptide-specific CTLs was judged to be positive when a significant value of p<0.05 was reached by a two tailed Student's t-test wand when the difference in IFN-γ production compared to the HIV peptide was more than 50pg/ml.

### 1.4 Peptides.

Forty two prostate cancer protein relating peptides including ten PSA-derived peptides, twelve PAP-derived peptides, and twenty PSMA-derived peptides were prepared based on the binding motifs to the HLA-A3, -A11, - A31, -A33 and -A68.1 molecules (Parker KC, Bednarek MA, Cokigan JE., J Immunol 1994;152:163-75.). Although these 5 HLA-A alleles share binding motifs, we preferentially considered the binding capacity to HLA-A11, -A31, and -A33 molecules, because HLA-A3⁺ or HLA-A68.1⁺ Japanese are very rare. Amino acid sequences of those peptides are shown in Table 1 below.

**Table 1 Summary of prostate-rented antigen-derived peptide candidates binding to the HLA-A3 supertype alleles**

| Peptides | Seqence | SEQ ID No. | Bind 10^{b} | Binding Score^{a} | | | | |
|---|---|---|---|---|---|---|---|---|
| | | | | A3 | A11 | A31 | A33 | A68.1 |
| PSA | | | | | | | | |
| 104-112 | ELLKNRFLR | 1 | | 18.0 | 0.4 | 12.0 | 9.0 | 10.0 |
| 60-68 | VLTAAHCIR | 2 | | 4.0 | 0.1 | 2.0 | 9.0 | 5.0 |
| 242-250 | LYTKWHYR | 3 | | 0.0 | 0.1 | 1.2 | 15.0 | 0.5 |
| 16-24 | GAAPULSR | 4 | | 0.5 | 0.2 | 0.8 | 3.0 | 15.0 |
| 241-250 | SLYTKVVHYR | 5 | | 90.0 | 0.2 | 12.0 | 9.0 | 5.0 |
| 59-68 | WVLTAAHCIR | 6 | | 0.6 | 0.6 | 4.0 | 15.0 | 400.0 |
| 36-45 | QPWQVLVASR | 7 | | 0.6 | 0.1 | 1.2 | 3.0 | 5.0 |
| 192-201 | VTKFMLCAGR | 8 | | 0.2 | 0.2 | 1.0 | 3.0 | 50.0 |
| 100-109 | LYDMSLLKNR | 9 | | 0.0 | 0.0 | 0.6 | 15.0 | 0.5 |
| 68-77 | RNKSVILLGR | 10 | | 0.0 | 0.0 | 0.5 | 0.9 | 1.0 |

| PAP | | | | | | | | |
|---|---|---|---|---|---|---|---|---|
| 20.28 | FLLFFWLDR | 11 | | 36.0 | 0.2 | 8.0 | 9.0 | 10.0 |
| 324-332 | EYFVEMYYR | 12 | | 0.0 | 0.1 | 6.4 | 45.0 | 3.0 |
| 39-47 | TLVFRHGDR | 13 | | 1.8 | 0.1 | 4.0 | 9.0 | 10.0 |
| 78-86 | HYELGEYIR | 14 | | 0.0 | 0.2 | 3.0 | 15.0 | 0.8 |
| 155-163 | YLPFRNCPR | 15 | | 4.0 | 0.1 | 2.0 | 9.0 | 5,0 |
| 96-105 | SYKHEQVYIR | 16 | | 0.0 | 0.2 | 6.0 | 15.0 | 0.5 |
| 171-180 | TLKSEEFQKR | 17 | | 12.0 | 0.1 | 4.0 | 9.0 | 5.0 |
| 19-28 | LFLLFFWLDR | 18 | | 0.0 | 0.1 | 2.4 | 3.0 | 1.0 |
| 38-47 | VTLVFRHGDR | 19 | | 0.1 | 0.3 | 2.0 | 3.0 | 100.0 |
| 102-111 | VYIRSTDVDR | 20 | | 0.0 | 0.1 | 1.2 | 15.0 | 1.5 |
| 154-163 | LYLPFRNCPR | 21 | | 0.0 | 0.1 | 1.2 | 15.0 | 1.5 |
| 248-257 | GIHKQKEKSR | 22 | | 0.6 | 0.1 | 1.0 | 15.0 | 7.5 |

| PSMA | | | | | | | | |
|---|---|---|---|---|---|---|---|---|
| 590-596 | SIVLPFOCR | 23 | | 2.7 | 0.1 | 8.0 | 15.0 | 10.0 |
| 173-181 | OLVYVNYAR | 24 | | 8.1 | 0.1 | 6.0 | 27.0 | 30.0 |
| 199-207 | KIVIARYGK | 25 | | 27.0 | 3.6 | 6.0 | 0.2 | 6.0 |
| 680-688 | GLPDRPFYR | 26 | | 36.0 | 0.7 | 6.0 | 9.0 | 5.0 |
| 370-378 | RYVILGGHR | 27 | | | 0.4 | 3.6 | 4.5 | 1.5 |
| 455-463 | SIEGNYTLR | 28 | | 0.6 | 0.1 | 2.0 | 15.0 | 5.0 |
| 403-411 | GTLKKEGWR | 29 | | 0.3 | 0.9 | 2.0 | 3.0 | 150.0 |
| 247-255 | NLPGGGVQR | 30 | | 6.0 | 0.1 | 2.0 | 9.0 | 5.0 |
| 641-649 | EIASKFSER | 31 | | 0.4 | 0.0 | 1.2 | 45.0 | 30.0 |
| 207-215 | KVFRGNKVK | 32 | | 15.0 | 6.0 | 0.9 | 0.2 | 240.0 |
| 354-363 | RIYNVIGTLR | 33 | | 3.0 | 0.5 | 18.0 | 4.5 | 5.0 |
| 181-190 | RTEDFFKLER | 34 | | 1.2 | 1.2 | 6.0 | 0.9 | 50.0 |
| 675-664 | FIDPLGLPDR | 35 | | 0.9 | 0.1 | 4.0 | 15.0 | 7.5 |
| 525-534 | FQRLGIASGR | 36 | | 0.2 | 0.1 | 2.0 | 3.0 | 5.0 |
| 345-354 | HIHSTNEVTR | 37 | | 0.4 | 0.1 | 2.0 | 15.0 | 7.5 |
| 201-210 | VIARYGKVFR | 38 | | 0.4 | 0.1 | 2.0 | 15.0 | 10.0 |
| 361-370 | TLRGAVEPDR | 39 | | 9.0 | 0.1 | 2.0 | 9.0 | 7.5 |
| 571-580 | KYHLTVAQVR | 40 | | 0.0 | 0.2 | 1.8 | 4.5 | 0.5 |
| 272-281 | YPANEYAYRR | 41 | | 0.4 | 0.1 | 1.0 | 3.0 | 10.0 |
| 431-440 | STEWAEEMSR | 42 | | 0.2 | 0.2 | 1.0 | 3.0 | 75.0 |
| EBV | IVTDFSVIK | 43 | A11 | 10.0 | 4.0 | 0.6 | 0.5 | 240.0 |
| Flu | NVKNLYEKVK | 44 | A11 | 3.0 | 1.0 | 0.1 | 0.5 | 180.0 |
| TRP2 | LLGPGRPYR | 45 | A31/A33 | 6.0 | 0.1 | 2.0 | 9.0 | 15.0 |
| HIV | RLRDLLLIVTR | 46 | A31 | | | | | |

| | | | | | | | | |
|---|---|---|---|---|---|---|---|---|
| ^{a}The peptide binding score was calculated based on the predicted half-time of dissociation. ^{b}Previously reported HLA class I alletes in which the peptides have immunogenicity are shown. | | | | | | | | |

All peptides were of >90% purity and were purchased from the Biologica Co. (Nagoya, Japan). Influenza (Flu) virus-derived (NVKNLYEKVK), Epstein-Barr virus (EBV)-derived (AVFDRHSDAH), tyrosinase-related protein 2 (TRP2) -derived (LLGPGRPYR), and HIV-derived (RLRDLLLIVTR) peptides were used as controls binding to HZA-A3 supertype alleles. All peptides were dissolved with dimethyl sulfoxide at a dose of 10 µg/ml.

### 1.5 Cytotoxicity Assay.

Peptide-stimulated PBMCs were tested for their cytotoxicity against LNCaP, LNCaP-A11, LNCaP-A31, or LNCaP-A33 by a standard 6-hr ⁵¹Cr-release assay. Phytohemagglutinin (PHA)-activated T cells were used as a negative control. Two thousand ⁵¹Cr-labeled cells per well were cultured with effector cells in 96-round-well plates at the indicated effector/target ratio. Immediately before the cytotoxicity assay, CD8⁺ T cells were isolated using a CD8 Positive Isolation Kit (Dynal, Oslo, Norway). The specific ⁵¹Cr-release was calculated according to the following formula: (test c.p.m.- spontaneous c.p.m.). Spontaneous release was determined by the supernatant of the sample incubated with no effector cells, and the total release was then determined by the supernatant of the sample incubated with 1% Triton X (Wako Pure Chemical Industries, Osaka, Japan). In some experiments, 10 µg/ml of either anti-HLA-class I (W6/32: mouse IgG2a), anti-HLA-class II (HLA-DR) (L243: mouse IgG2a), or anti-CD14 (H14: mouse IgG2a) mAb was added into wells at the initiation of the culture.

### 1.6 Cold Inhibition Assay.

The specificity of peptide-stimulated CTLs was confirmed by the cold inhibition assay. Briefly, ⁵¹Cr-labeled target cells (2 × 10³ cells/well) were cultured with the effector cells (2 × 10⁴cells/well) in 96-round-well plates with 2 × 10⁴ cold target cells. C1R-A11, -A31, and -A33, which were pre-pulsed with either the HIV peptide or a corresponding peptide, were used as cold target cells.

**1.7 Detection of Peptide-Specific IgG.**
Peptide-specific IgG levels in the plasma were measured by ELISA as previously reported (Nakatsura T, Senju S, Ito M, Nishimura Y, Itoh K., Eur J Immunol 2002;32:826-36.). Briefly, a peptide (20 µg/well)-immobilized plate was blocked with Block Ace (Yukijirushi, Tokyo, Japan), and 100 µl/well of plasma sample diluted with 0.05% Tween-20-Block Ace was added to the plate. After a 24-hr incubation at 4°C, the plate were washed and further incubated for 2 hr with a 1:1000-diruted rabbit anti-human IgG (γ-chain-specific) (Dako, Glostrup, Denmark). The plate was washed, and then 100 µl of 1:100-diluted goat anti-rabbit IgG-conjugated horseradish peroxidase (En Vision; Dako) was added to each well, and the plate was incubated at room temperature for 40 min. After the plate was washed again, 100 µl/well of tetramethyl benzidine substrate solution (KPL, Guildford, UK) was added, and the reaction was stopped by the addition of 1 M phosphoric acid. To estimate peptide-specific IgG levels, we compared the optical density (OD) values of each sample with those of serially diluted samples, and the values are shown as OD U/ml. IgG reactive to the corresponding peptide was judged to be significant when the OD in 1:100-diluted plasma exceeded the mean + 3SD of the OD of the IgG reactive to the control, HIV peptide. To confirm the specificity of IgG reactive to relevant peptides, samples were cultured in peptide-coated plates, and the levels of peptide-specific IgG in the supernatant were determined by ELISA.

### 1.8 Statistics.

The statistical significance of the data was determined using a two-tailed Student's t-test. A P value of less than 0.05 was considered statistically significant.

### 2. RESULTS

### 2.1 Detection of IgG Reactive to the PSA, PAP, and PSMA Peptides.

In this study, we first screened 42 peptide candidates based on their ability to be recognized by IgGs of prostate cancer patients. We did this type of screening because it seemed very difficult to perform an in vitro sensitization experiment using 42 peptides, and because we have observed that IgGs reactive to CTL-directed peptides are frequently detected in the plasma of patients with several types of cancers. Results are shown in Table 2.

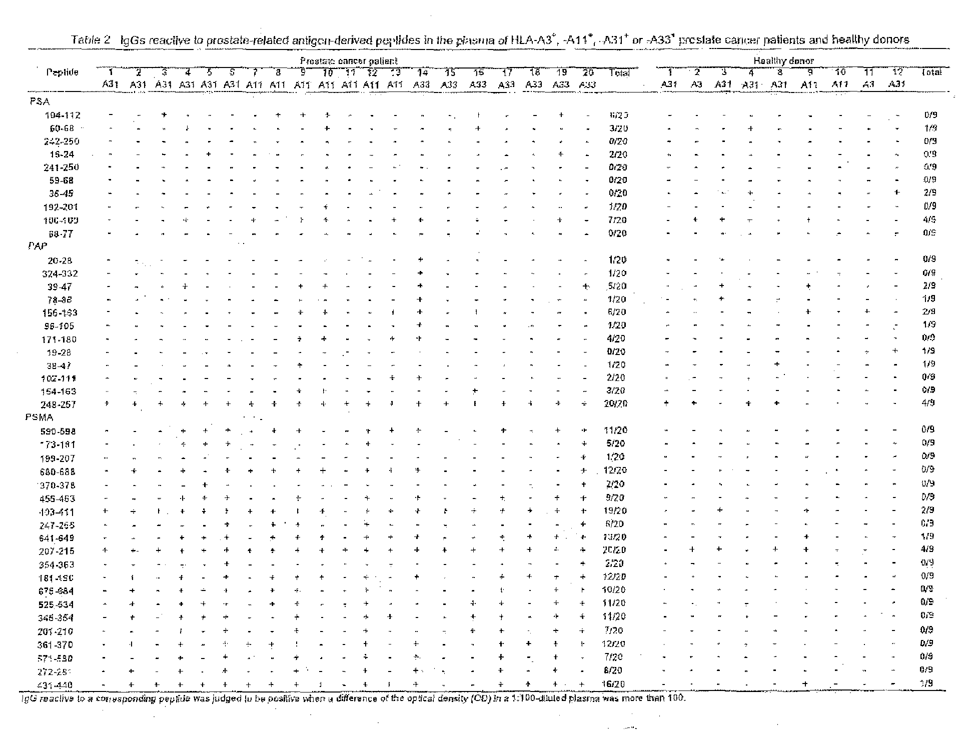

IgGs reactive to either the PSA ₁₀₄₋₁₁₂ PSA ₆₀₋₆₈ PSA ₁₆₋₂₄. or PSA 100-109 peptide were detected in the plasma of prostate cancer patients more frequently among the IgGs reactive to the PSA peptides. IgGs reactive to either the PAP 39-47, PAP 155-163, PAP 171-180, or PAP 248-257 peptide were detected in the plasma of prostate cancer patients more frequently among the IgGs reactive to the PAP peptides. IgGs reactive to either the PSMA ₄₀₃₋₄₁₁, PSMA ₆₄₁₋₆₄₉, PSMA ₂₀₇₋₂₁₅, or PSMA ₄₃₁₋₄₄₀ peptide were detected in the plasma of prostate cancer patients more frequently among the IgGs reactive to the PSMA peptides. It seems that peptide-specific IgG was more frequently detected in cancer patients than in healthy donors.

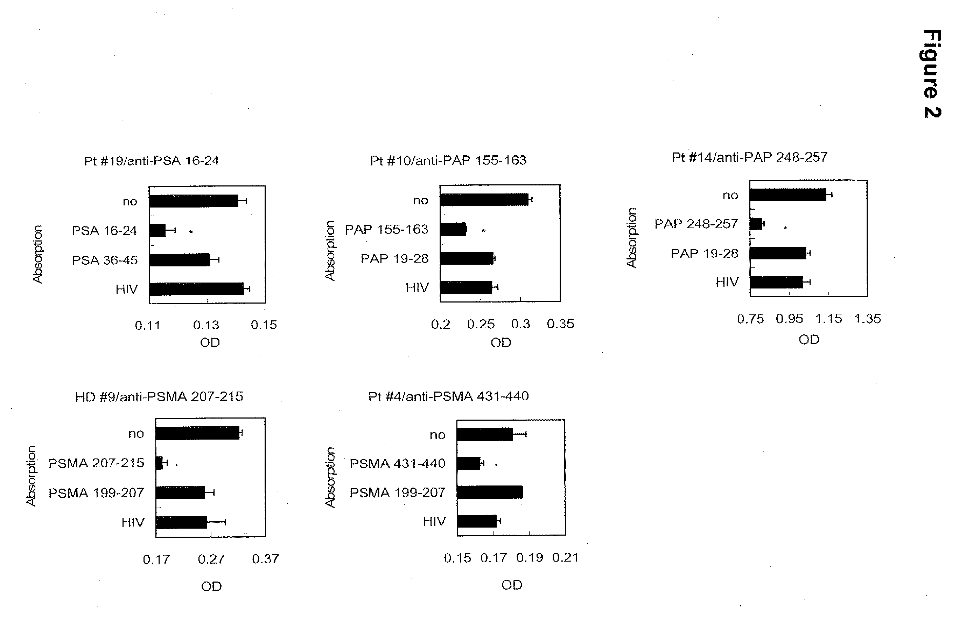

We further confirmed the validity of the assay of peptide-specific IgGs. Representative results are shown in Figure 2. The levels of IgGs reactive to PSA₁₆₋₂₄, PAP₁₅₅₋₁₆₃, PAP₂₄₈₋₂₅₇, PSMA₂₀₇₋₂₁₅, and PSMA₄₃₁₋₄₄₀ peptides in the plasma of patient were significantly diminished by incubating the samples in wells that were coated with each of the corresponding peptides. This result indicates this study is reliable for detecting peptide-specific IgG.

### 2.2 Induction of Peptide-Specific CTLs from the PBMCs of Prostate Cancer Patients.

We determined whether or not peptide candidates that were frequently recognized by IgGs in cancer patients could induce peptide-specific CTLs from the PBMCs of HLA-A11⁺, -A31⁺, and -A33⁺ prostate cancer patients. The PSA 36-45, PAP 19-28, and PSMA 199-207 peptides were used as negative controls that were recognized by IgG less frequently. Positive results are summarized in Table 3.

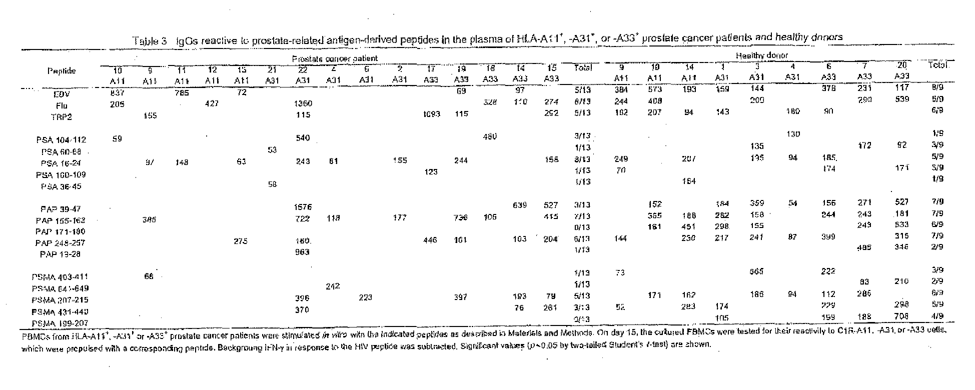

The PSA₁₆₋₂₄, PAP₁₅₅₋₁₆₃, PAP₂₄₈₋₂₅₇, PSMA₂₀₇₋₂₁₅, and PSMA₄₃₁₋₄₄₀ peptides induced corresponding peptide-reactive CTLs from the PBMCs of 3, 1, 1, 0, and 0 out of 5 HLA-A11⁺ cancer patients, 3, 3, 1, 2, and 1 out of 5 HLA-A31⁺ cancer patients, and 2, 3, 4, 3, and 2 out of 5 HLA-A33⁺ cancer patients, respectively (Table 3). These peptides also effectively induced peptide-specific CTLs from the PBMCs of healthy donors. These findings indicate that the PSA₁₆₋₂₄, PAP₁₅₅₋₁₆₃, PAP₂₄₈₋₂₅₇, PSMA₂₀₇₋₂₁₅, and PSMA₄₃₁₋₄₄₀ peptides are useful for generating peptide-specific CTLs in the PBMCs of prostate cancer patients with HLA-A3 supertype alleles.

### 2.3 Induction of Prostate Cancer-Reactive CTLs from the PBMCs of Prostate Cancer Patients with HLA-A3 Supertype Alleles.

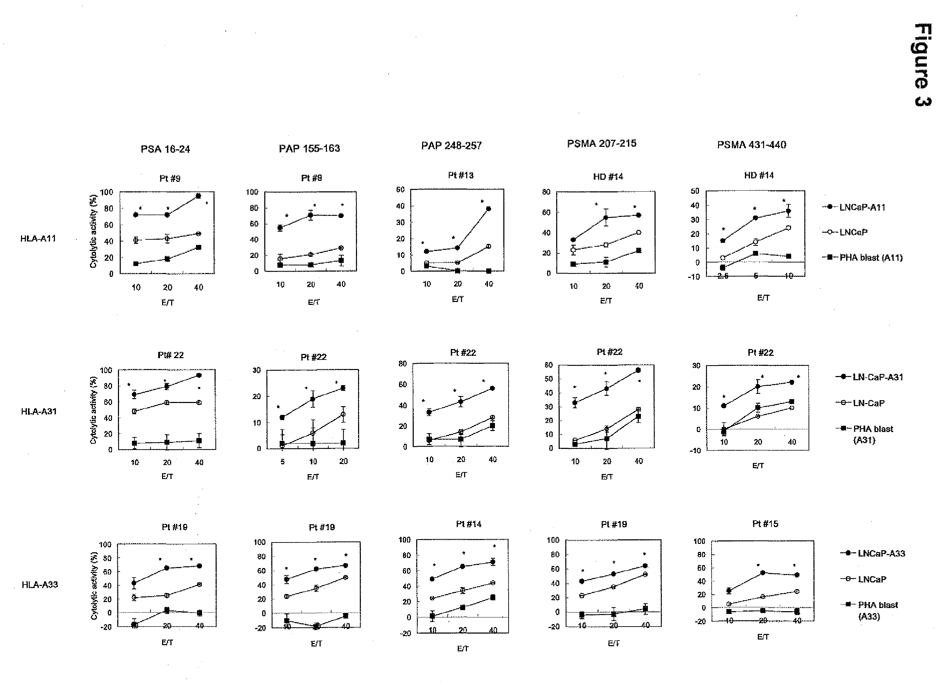

We determined whether or not CTLs induced by *in vitro* stimulation with each of the PSA₁₆₋₂₄, PAP₁₅₅₋₁₆₃, PAP₂₄₈₋₂₅₇, PSMA₂₀₇₋₂₁₅, and PSMA₄₃₁₋₄₄₀ peptides could show cytotoxicity against prostate cancer cells. The PBMCs from two HLA-A11⁺ patients (#9 and #13) and one healthy donor (#14), one HLA-A31⁺ patient (#22), and three HLA-A33⁺ patients (#14, #15 and #19) were stimulated with the peptides indicated in the table and it was determined whether or not peptide-reactive CTLs from the HLA-A11⁺ patients, HLA-A31⁺ patients, and HLA-A33⁺ patients could show cytotoxicity against prostate cancer cells expressing the HLA-A11, HLA-A31, and HLA-A33 molecules, respectively. Results are shown in (Figure 3). The PBMCs from HLA-A11⁺ patients and from healthy donor, which were stimulated in vitro with each of the PSA₁₆₋₂₄, PAP₁₅₅₋₁₆₃, PAP₂₄₈₋₂₅₇, PSMA₂₀₇₋₂₁₅, and PSMA₄₃₁₋₄₄₀ peptides, exhibited a higher level of cytotoxicity against LNCaP-A11 than against LNCaP and HLA-A11⁺ T-cell blasts. Similarly, these peptides possessed the ability to induce prostate cancer-reactive CTLs from PBMCs of HLA-A31⁺ patient and HLA-A33⁺ patients. Those results indicate that the PBMCs stimulated *in vitro* with the PSA₁₆₋₂₄, PAP₁₅₅₋₁₆₃, PAP₂₄₈₋₂₅₇, PSMA₂₀₇₋₂₁₅, or PSMA₄₃₁₋₄₄₀ peptide could show cytotoxicity against prostate cancer cells in an HLA-A11, -A31, or -A33-restricted manner.

### 2.4 Peptide-Specific and CD8⁺T Cell-Dependent Cytotoxicity against Prostate Cancer Cells.

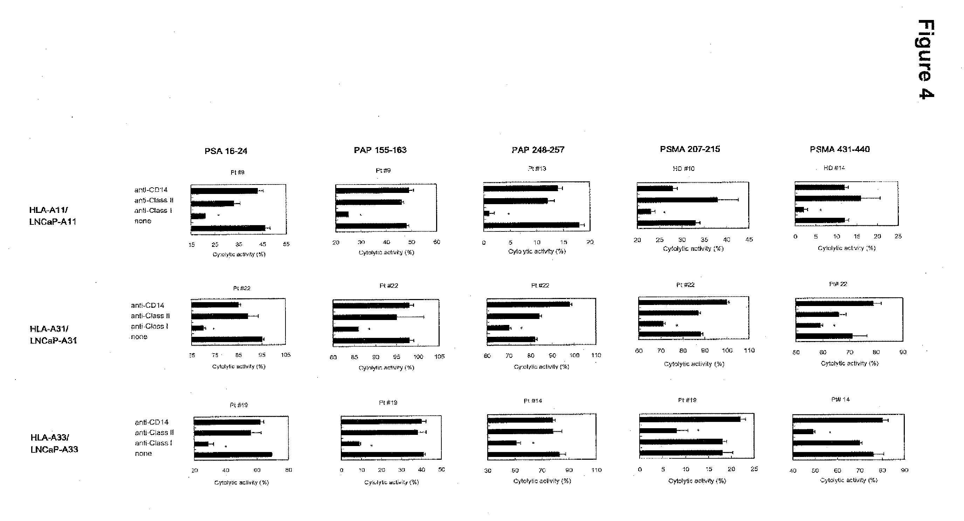

We tried to identify cells that were responsible for the cytotoxicity of peptide-stimulated PBMCs. As shown in Figure 4, the cytotoxicity of the PSA ₁₆₋₂₄, PAP ₁₅₅₋₁₆₃, PAP ₂₄₈₋₂₅₇, PSMA ₂₀₇₋₂₁₅, or PSMA ₄₃₁₋₄₄₀ peptide-stimulated CD8⁺T cells from HLA-A11⁺ patients (#9 and #13), HLA-A11⁺ healthy donors (#10 and #14), HLA-A31⁺ patient (#22), and HLA-A33⁺ patients (#19 and #14) were significantly inhibited by the addition of anti-HLA class I mAb, but not by the addition of anti-HLA class II (HLA-DR) or anti-CD14 mAb. These results indicated that the cytotoxicity of peptide-stimulated PBMCs against prostate cancer cells was dependent on HLA class I-restricted CD8⁺ T cells.

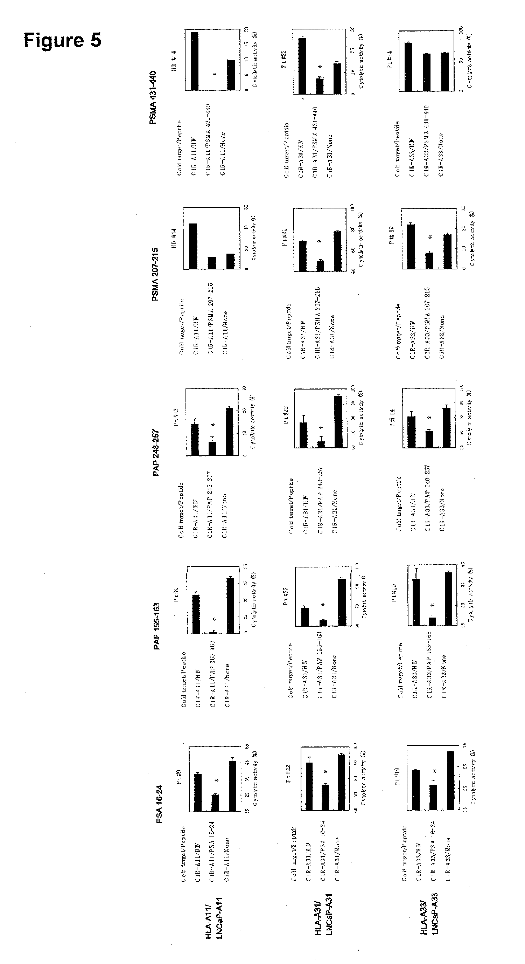

In addition, their cytotoxicity against LNCaP-A11, -A31, and -A33 was significantly suppressed by the addition of corresponding peptide-pulsed unlabeled C1R-A11, -A31, and -A33 cells, but not by HIV peptide-pulsed unlabeled C1R-A11, -A31, or -A33 cells (Figure 5). However, no such inhibition was observed in two cases: the PBMCs of healthy donor #14 and patient #14 which were respectively stimulated with the PSMA ₂₀₇₋₂₁₅ and PSMA ₄₃₁₋₄₄₀ peptides. Nevertheless, the results as a whole indicate that the cytotoxicity of peptide-stimulated PBMCs against prostate cancer cells could be ascribed to the corresponding peptide-specific CD8⁺ T cells.

### 2.5 Cytotoxicity of Peptide-Stimulated PBMCs against Prostate Cancer Cells Sharing HLA-A3 Supertype Alleles.

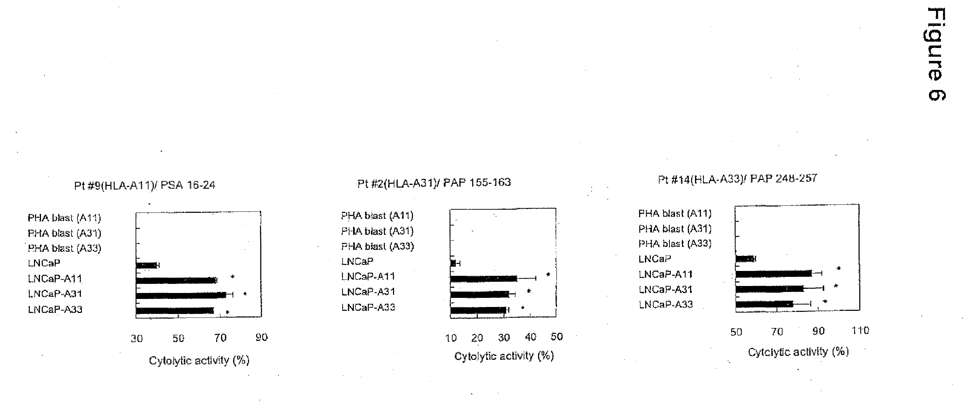

We determined whether or not peptide-stimulated PBMCs positive for one of the HLA-A3 supertype alleles could show cytotoxicity against prostate cancer cells expressing other alleles of the HLA-A3 supertype. As shown in Figure 6, the PBMCs from patients #9(HLA-A11⁺), #2 (HLA-A31⁺) and #22(HLA-A33⁺), which were stimulated in vitro with the PSA ₁₆₋₂₄, PAP ₁₅₅₋₁₆₃, and PAP ₂₄₈₋₂₅₇ peptides, respectively, showed higher levels of cytotoxicity against LNCaP-A11, LNCaP-A31, and LNCaP-A33 cells compared to LNCaP cells. The cytotoxicity against PHA-stimulated T-cell blasts was not observed. These results indicate that peptide-stimulated PBMCs could show cytotoxicity against prostate cancer cells sharing HLA-A3 supertype alleles. These results suggest that the peptide of the invention are applicable to prostate cancer patients positive for any of the HLA-A3 supertype alleles.

## Claims

1. A peptide, which is a prostate-related protein derived peptide, capable of binding to an HLA-A3 supertype allele and recognized by the cellular and/or humoral immune system.

2. The peptide of Claim 1, which is recognized by both the cellular and humoral immune systems.

3. The peptide of Claim 1or 2, which is consisting of 8-11 amino acid residues.

4. The peptide of any one of Claims 1-3, which has an amino acid sequence shown in any one of SEQ ID NOS: 4, 15, 22, 32 and 42.

5. A derivative of the peptide of Claim 4, which has the functionally equivalent properties.

6. The derivative of Claim 5, which has an amino acid sequence shown in any one of SEQ ID NOS:4, 15, 22, 32 and 42 whose C-terminus amino acid is substituted by lysine or arginine.

7. A nucleic acid molecule encoding the peptide of any one of Claims 1-4 or the peptide derivative of Claim 5 or 6.

8. A vector comprising the nucleic acid molecule of Claim 7.

9. A pharmaceutical composition comprising the peptide of any one of Claims 1-4, the peptide derivative of Claim 5' or 6, or the vector of Claim 8 for the treatment or prevention of prostate cancer.

10. The pharmaceutical composition of Claim 9, which is a cancer vaccine comprising a peptide having an amino acid sequence shown in any one of SEQ ID NOS:4, 15, 22, 32 and 42.

11. A method for the treatment or prevention of prostate cancer in a subject, which comprises administering the peptide of any one of Claims 1-4, the peptide derivative of Claim 5 or 6, or the vector of Claim 8 to the subject.

12. The method of Claim 11, which comprises administering a peptide having an amino acid sequence shown in any one of SEQ ID NOS:4, 15, 22, 32 and 42 as a cancer vaccine to the subject.

13. Use of the peptide of any one of Claims 1-4, the peptide derivative of Claim 5 or 6, or the vector of Claim 8 for manufacturing a pharmaceutical composition for the treatment or prevention of prostate cancer.

14. Use of Claim 13, wherein the pharmaceutical composition is a cancer vaccine comprising a peptide having an amino acid sequence shown in any one of SEQ ID NOS:4, 15, 22, 32 and 42.

15. A method for inducing prostate cancer reactive cytotoxic T cells, which comprises the step of contacting peripheral blood mononuclear cells derived from an HLA-A3 supertype allele positive prostate cancer patient with the peptide of any one of Claims 1-4 or the derivative of Claim 5 or 6.

16. The method of Claim 15, which uses a peptide having an amino acid sequence shown in any one of SEQ ID NOS:4, 15, 22, 32 and 42.

17. A cytotoxic T cell induced by the method of Claim 15 or 16.

18. A kit for inducing prostate cancer reactive cytotoxic T cells, which comprises the peptide of any one of Claims 1-4 or the peptide derivative of Claim 5 or 6.

19. A method for the preparation of an antigen presenting cell on which a complex between a prostate related protein derived peptide or a derivative thereof and an HLA-A3 supertype allele is presented, which comprises allowing a cell having antigen-presenting ability derived from an HLA-A3 supertype allele positive prostate cancer patient to be incorporated with the peptide of any one of Claims 1-4, the peptide derivative of Claim 5 or 6, or the vector of Claim 8.

20. The method of Claim 19, wherein the peptide is the one having an amino acid sequence shown in any one of SEQ ID NOS:4, 15, 22, 32 and 42.

21. An antigen presenting cell prepared by the method of Claim 19 or 20.

22. A kit for the preparation of an antigen presenting cell on which a complex between a prostate related protein derived peptide or a derivative thereof and an HLA-A3 supertype allele is presented, which comprises the peptide of any one of Claims 1-4, the peptide derivative of Claim 5 or 6, or the vector of Claim 8.
